# EUROPEAN PATENT APPLICATION

(11) **EP 1 251 166 A1**
(43) Date of publication of application: **23.10.2002**
(21) Application number: 02252080.3
(22) Date of filing: 22.03.2002
(51) Int. Cl.: C12N 5/00

(54) **Method for selecting spermatozoon**

(30) Priority: 10.04.2001 IL 14253301
(71) Applicant: Bartoov, Benjamin, Moshav Hemed 50295 (IL)
(72) Inventor: Bartoov, Benjamin, Moshav Hemed 50295 (IL)
(74) Representative: Ablewhite, Alan James

(57) **Abstract**

The invention provides a method for individually selecting spermatozoa for intracytoplasmic sperm microinjection comprising effecting a real-time morphological evaluation of washed, fresh, motile spermatozoa without fixation using a high power light microscope having a magnification of at least x4000, selecting individual spermatozoon exhibiting high normalcy values for the morphological state of the nuclei of each individual spermatozoon and retrieving selected individual motile spermatozoon with nuclei exhibiting a smooth oval-shaped configuration, a length of between about 4.47 and 5.03 µm, and a width of about 3.08 and 3.48 µm.

## Description

The present invention relates to a method for selecting spermatozoa for intracytoplasmic sperm microinjection (hereinafter ICSI). More specifically, the present invention relates to a method utilizing real time morphological evaluation of washed, fresh, motile spermatozoa without fixation in order to achieve a functional morphology which will allow a higher pregnancy rate as a result of ICSI procedures.

In the recent authoritative text, The Value Of Sperm Morphology And Other Semen Parameters In Diagnosis And Treatment Of Human Subfertility by Willem Ombelet, published in 1998, there is described the methodological aspects of examination of human semen from 1951 until the present time.

Sperm morphology has been recognized to be the best predictor of outcome for natural fertilization [Bartoov,B., Eltes,F., Pansky,M., *et al*. (1994) Improved diagnosis of male fertility potential via a combination of quantitative ultramorphology and routine semen analyses. *Hum. Reprod*., **9**, 2069-2075, and Bartoov,B., Eltes,F., Pansky,M., *et al.* (1993) Estimating fertility potential via semen analysis data. *Hum. Reprod*., **8**, 65-70.], intrauterine insemination (IUI) [Berkovitz,A., Eltes,F., Sofer,Y., *et al.* (1999) ART success and *in vivo* sperm cell selection depend on the spermatozoa ultramorphological status. *Andrologia* **31**, 1-8.] and conventional in vitro fertilization (IVF) [Kruger,T.F., Acosta,A.A., Simmons,K.F., *et al*. (1988) Predictive value of sperm morphology in *in-vitro* fertilization. *Fertil. Stehl*., **49**, 112-117 and; Mashiach,R., Fisch,B., Eltes,F., *et al*. (1992) The relationship between sperm ultrastructural features and fertilization capacity *in vitro. Fertil. Steril.,* **57**, 1052-1057]. Surprisingly, however, according to a great number of investigators, it is not related to the success of IVF-ICSI. [Nagy,Z.P., Verheyen,G., Tournaye,H., *et al.* (1998) Special applications of intracytoplasmic sperm injection: the influence of sperm count, motility, morphology, source and sperm antibody on the outcome of ICSI. *Hum. Reprod*., **13** (Suppl. 1), 143-154.; Svalander,P., Jakobsson,A.H., Forsberg,A.S., *et al*. (1996) The outcome of intracytoplasmic sperm injection is unrelated to 'strict criteria' sperm morphology. *Hum. Reprod*., **11**, 1019-1022; and Oehninger,S., Chaturvedi,S., Toner,J., *et al*. (1998) Semen quality: is there a paternal effect on pregnancy outcome in *in-vitro* fertilization/intracytoplasmic sperm injection? *Hum. Reprod*., **13**, 2161-2164.] This finding may be explained by the methodology of ICSI itself in that it bypasses the barriers of the female tract and zona pellucida which require morphologically normal spermatozoa for good passage. [Van Steirteghem, A., Tourneys, H., Van der Elst, et al. (1995) Intracytoplasmic sperm injection three years after the birth of the first ICSI child. Review. Hum. Reprod., **10**, 2527-2528; Nikolettos,N., Kupker,W., Demirel, C., *et al.* (1999) Fertilization potential of spermatozoa with abnormal morphology. *Hum. Reprod.,* **14** (Suppl. 1), 47-70.] The lack of a relationship between sperm morphology and ICSI success may also involve the method of sperm selection for microinjection, wherein spermatozoa with severe head defects are usually excluded [Nikolettos,N., Kupker,W., Demirel,C., *et al*. (1999) Fertilization potential of spermatozoa with abnormal morphology. *Hum. Reprod.,* **14** (Suppl. 1), 47-70.]. Indeed, such spermatozoa, especially with round, large or tapered heads, have been reported to reduce fertilization, implantation, and pregnancy rates as well as to cause more intrauterine deaths [Tasdemir,I., Tasdemir,M.M., Tavukcuoglu,S., *et al*. (1997) Effect of abnormal sperm head morphology on the outcome of intracytoplasmic sperm injection in humans. *Hum. Reprod*., **6**, 1214-1217; ]

Nevertheless, the reported fertilization and pregnancy rates for ICSI are relatively low; about 65% and 30%, respectively. [Tarlatzis,B.C. and Bili,H. (1998) Survey on intracytoplasmic sperm injection: report from the ESHRE ICSI Task Force. European Society of Human Reproduction and Embryology. *Hum. Reprod*., **13** (Suppl. 1), 165-177.]

The present invention is based on the discovery that the outcome of ICSI is affected by subtle morphological sperm malformations that remain undetectable by embryologists who, according to accepted practice, select spermatozoa for microinjection under routine light microscopy magnification (×200-×400).

With this state of the art in mind, there is now provided according to the present invention, a method for individually selecting spermatozoa for intracytoplasmic sperm microinjection comprising effecting a real-time morphological evaluation of washed, fresh, motile spermatozoa without fixation using a high power light microscope having a magnification of at least x4000, selecting individual spermatozoon exhibiting high normalcy values for the morphological state of the nuclei of each individual spermatozoon and retrieving selected individual motile spermatozoon with nuclei exhibiting a smooth oval-shaped configuration, a length of between about 4.47 and 5.03 µm, and a width of about 3.08 and 3.48 µm.

In preferred embodiments of the present invention, said retrieved spermatozoon are further characterized by a nuclear chromatin content of less than 4 per cent vacuoles for the entire nuclear area.

In especially preferred embodiments of the present invention, said method further comprises evaluating the normalcy of the acrosome, postacrosomal lamina, neck, mitochondria, tail and nucleus subcellular organelles of each individual spermatozoon.

In preferred embodiments of the present invention, said evaluation is carried out using a high-power light microscope having a magnification of at least x4000.

Preferably, said retrieval is effected utilizing a micromanipulated transparent needle for aspirating said selected individual motile spermatozoon.

In preferred embodiments of the present invention said needle has a diameter of between about 15 and 20 microns and is preferably made of glass.

Thus, according to the present invention, there is now provided a new method for the detailed morphological evaluation of motile spermatozoa in real time hereinafter referred to as the motile sperm organelle morphology examination (MSOME). In the following examples, MSOME was applied only to the sperm fraction routinely preselected for ICSI. It was performed with a high powered (×4000 - ×6300) inverted light microscope equipped with Nomarski optics and a CCD camera.

The invention will now be described in connection with certain preferred embodiments in the following examples and with reference to the following illustrative figures so that it may be more fully understood.

With specific reference now to the figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
**Figures 1a and 1b** are high power light microscope micrographs of motile sperm
**Figure 2.** Is a graphic representation of the distribution of fertilization rate in the examined population.
**Figure 3a.** Is a graphic representation of the morphologically normal spermatozoa:
**Figure 3b.** Is a graphic representation of spermatozoa with morphologically normal nucleus.
**Figure 4a.** Is a graphic representation of predictive values of morphologically normal spermatozoa (---) and spermatozoa with morphologically normal nucleus (―) for ICSI fertilization rate.
**Figure 4b.** Is a graphic representation of predictive value of spermatozoa with morphologically normal nucleus for achieving pregnancy following ICSI and .
**Figure 5.** Is a graphic representation of distribution of spermatozoa with morphologically normal nucleus in the successful and failed pregnancy subgroups (AV-average value ± SE).

### EXAMPLES

### MATERIALS AND METHODS

### Patients

The study population consisted of the male partners of 100 random couples referred for ICSI treatment at two major IVF units in Israel (Rabin and Herzilya Medical Centers, n=90) or a major IVF unite at the Institut Rhonalpin, Laboratoire Marcel Merieux in Bron (France, n=10). Inclusion criteria were: female partner younger than 38 years; and ≥3 retrieved ova per cycle. The mean age of the males was 34.9±4.1 years (range 27-41 years) and of the females, 30.3±5.0 years (range 22-38 years).

### ICSI procedure

Indications for ICSI in the three IVF centers were as follows: 1. poor (< 20%) or no (0%) fertilization in the first IVF cycle; 2. immotile spermatozoa; 3. low percentage of normally formed spermatozoa [< 5% according to the definition of Kruger et al. (1988 ibid) or < 30% according to the (World Health Organization (WHO) (1992) [*Laboratory Manual for the Examination of Human Semen and Semen-Cervical Mucus Interaction, 3*^{*rd*} *edition*. Cambridge University press, Cambridge, UK.)]. The ICSI procedure was performed according to the method described by Palermo,G.D., Joris,H., Devroey,P., *et al.* (1992) Pregnancy after intracytoplasmic injection of single spermatozoa into an oocyte. *Lancet*, **340**, 17-18.

Fertilization was recorded if two pronuclei could be detected after 12 to 16 hours and, finally, if cleavage occurred. A 60% fertilization rate was defined as normal; rates below 60% were defined as low. This cutoff was established prior to onset of the study on the basis of the general IVF-ICSI outcome data obtained from the three participating IVF centers.

Embryo transfer was usually performed on day 3; on Saturdays and holidays in Israel, it was sometimes performed on day 2. Pregnancy was confirmed at six to seven weeks by ultrasound examinaiton. The pregnancy rate was computed by dividing the number of pregnancies by the number of successful embryo transfers.

### Sperm preparation for morphological observation

After completing the ICSI procedure, unused spermatozoa were transported to the Male Fertility Laboratory of Bar-Ilan University for MSOME. The 90 sperm samples from the Israeli IVF units arrived to the laboratory within a few hours after microinjection, and the 10 samples from France were sent by special delivery and arrived after 24-48 hours. As no statistically significant difference was noted in the distribution of the morphological sperm characteristics between the Israeli and French semen samples, all 100 samples were analyzed together.

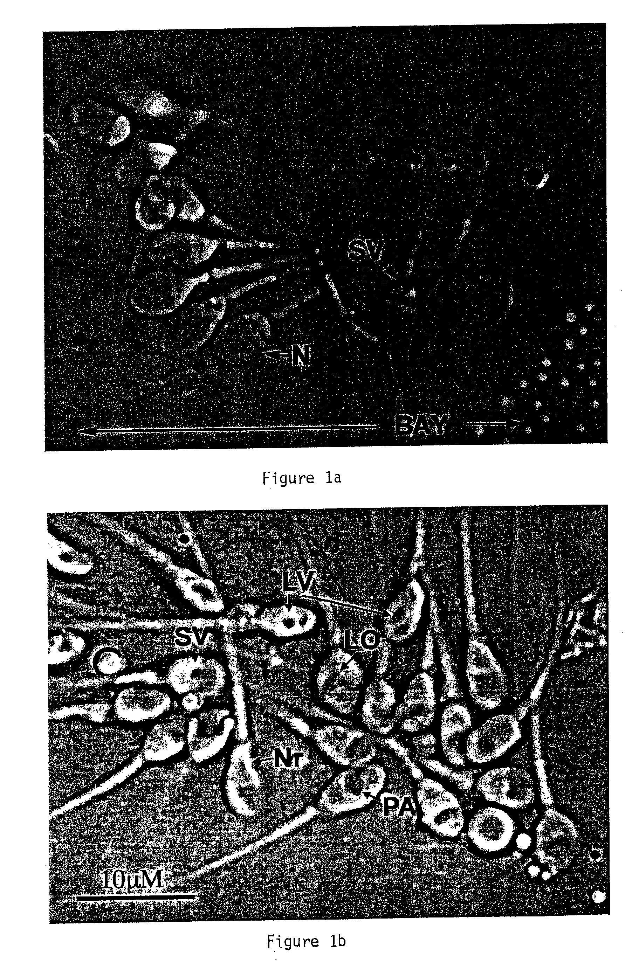

The fraction of unused spermatozoa, usually suspended in IVF medium (MEDI-CULT, Mollehaven 12, 4040 Jyllinge, Denmark) or Sperm medium (MEDI-CULT) was mixed. An aliquot of 1-2 µl of the sperm suspension containing a few thousand spermatozoa was transferred to a micro-droplet of Sperm medium containing 0-8% polyvinyl pyrrolidone (PVP) solution (MEDI-CULT PVP Medium Cat. No. 10890001) and placed in a sterile WillCo glass-bottomed dish (BioSoft International, B.V. Art. No. GWSt-1000) under sterile paraffin oil (OVOIL-150, Science, Scandinavia). The temperature of the observed sample and the PVP concentration were coordinated with the intensity of the sperm motility. In cases of poor sperm motility, the temperature was elevated to 37°C, no PVP was added to the Sperm medium in the recipient droplet, and the sperm suspension medium was supplemented with 6% human serum albumin (KAMADA LTD, Kibbutz Beit Kama, Israel). In cases of high sperm motility, the temperature was lowered to about 20°C and the PVP concentration was kept high (about 8%). Morphological assessment of the sperm cells in motion was made possible by the creation of small bays extruding from the rim of the droplets, which captured the heads of the motile spermatozoa (Fig. 1a).

### Sperm observation

The motile sperm fraction selected for ICSI was examined under immersion oil by an inverted microscope (Olympus IX 70) equipped with Nomarski optics, an Uplan Apo X 100/1.35 objective lens, and a 0.55NA condenser lens. The images were captured by a DXC-950P color video camera (Sony), which has a ½-inch, 3-chip power HAD CCD containing some 380000 effective picture elements (pixels) for high-quality image production, and a color video monitor (Sony PVM-14M4E, HR-Trinitron). The morphological assessment was conducted on the monitor screen which, under the above configuration, reached a real magnification of X6300, as determined by 0.01 mm Olympus objective micrometer. Calculation of the total magnification on the TV monitor was based on four parameters: 1.objective magnification 100x; 2. magnification selector 1.5x; 3, video coupler magnification 0.99 (UPMTVx0.3, PEx3.3); 4. (a) CCD chip diagonal dimension 8mm and (b) TV monitor diagonal dimension 355.6 mm, for a calculated video magnification (b/a) of 44.45. Thus, total magnification = microscope magnification (x150) x video coupler magnification (x0.99) x video magnification (x44.45) = 6600x.

It should be stressed that MSOME was applied exclusively to motile spermatozoa, which under low light microscopy magnification have a high potential to be selected for ICSI. Sperm cells with a severe malformation, such as pin, amorphous, tapered, round or multinucleated head, which can be clearly identified even by low magnification (×200-×400), were not assessed in this study, since such spermatozoa are routinely eliminated from injection into the oocyte.

One hundred motile spermatozoa from each sperm sample were examined for the morphological state of six subcellular organelles: acrosome, postacrosomal lamina, neck, mitochondria, tail and nucleus. The first five of these were defined as morphologically normal or abnormal on the basis of the presence of the specific malformations as listed in Table I hereinafter.

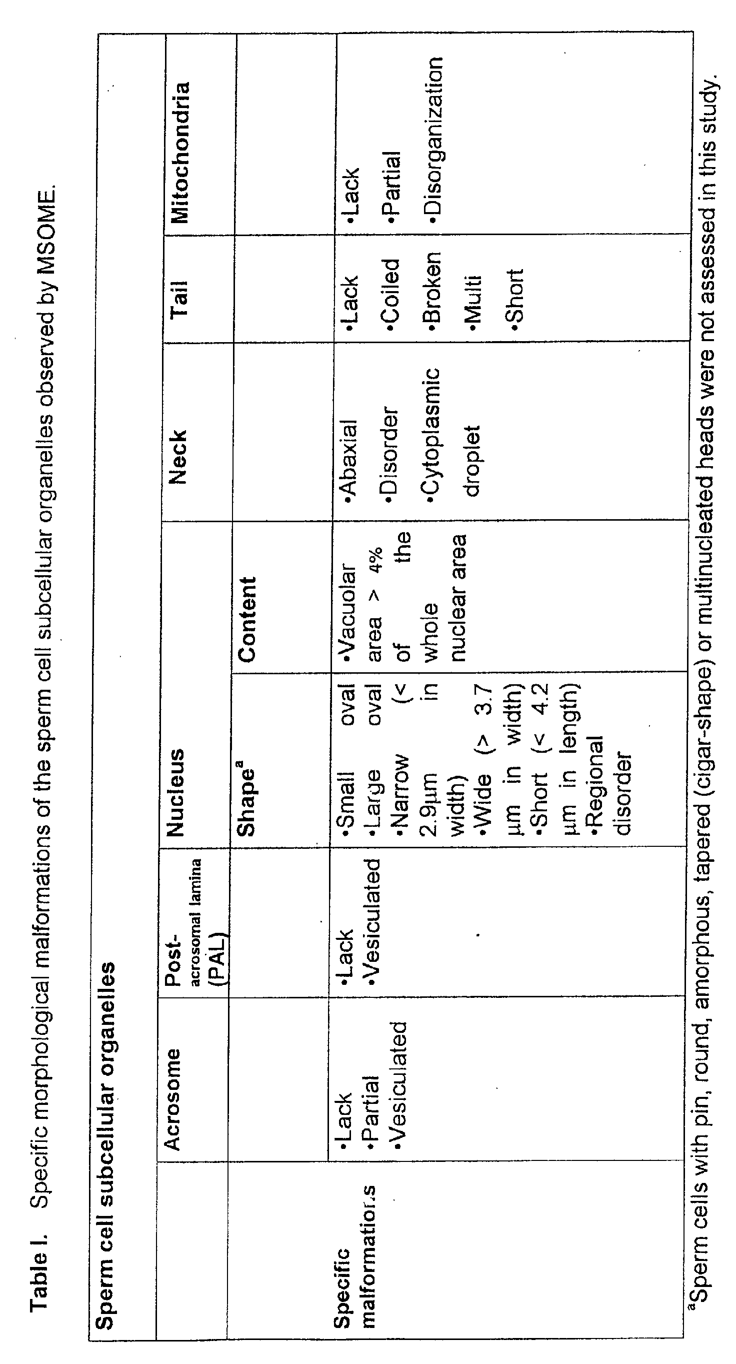

For the nucleus, the morphological state was defined by both shape and chromatin content. The criteria for normal shape were smooth configuration, oval shape, length 4.75±0.28µm, and width 3.28±0.20µm. To enable rapid evaluation of the nuclear shape, a fixed, transparent, celluloid form of a sperm nucleus fitting these criteria was superimposed on the examined cell; the nuclear shape was documented as abnormal if it veered in length or width by two standard deviations from the normal mean axes values (Fig. 1a, b).

Referring to said figures, the sperm heads were captured in small bays (BAY) extruding from the rim of the droplets. N-morphologically normal spermatozoa; VL-vesiculated postacrosomal lamina; LV-large nuclear vacuoles, SV-small nuclear vacuoles; LO-large oval sperm cells; Nr-spermatozoa with a narrow post-acrosomal region; PA-partial acrosome.

An extrusion or invagination of the nuclear chromatin mass was defined as a regional nuclear shape malformation (Table I). The nuclear chromatin content was considered abnormal if it contained one or more vacuoles that occupied more than 4% of the normal nuclear area (Fig. 1a, b). To be considered morphologically normal, a sperm nucleus had to have both a normal shape and normal chromatin content. A sperm cell exhibiting a normal nucleus as well as a normal acrosome, postacrosomal lamina, neck, tail, mitochondria and no cytoplasmic droplet or cytoplasm around the head was classified as morphologically normal (Fig. 1a, b).

The relationship between normal spermatozoa obtained by the routine method (WHO, 1992 ibid.) and by MSOME was assessed in 20 of the 100 examined patients. No correlation of the results was found. The incidence of sperm normalcy by routine sperm analysis was significantly higher than that by MSOME (26.1±7.2%, range: 0-68% and 2.9±0.5%, range: 0-5%, respectively, F=38.2, p≤0.01). It is noteworthy that the routine morphological examination is applied to the whole semen sample post-fixation wherein MSOME concentrates only on the fresh, unfixed motile sperm fraction.

### MSOME quality control

To determine intratechnician variability in the assessment of sperm morphology by MSOME, motile sperm cell fractions were obtained from five randomly selected patients referred to our laboratory for this procedure. Each sample was observed five times (total, 5x5 observations) by the same observer (B.B.), who was blinded to the source of the 25 samples, and the correlation among the five observations was analyzed using Cronbach's alpha of reliability. A high Cronbach's alpha value was obtained for 9 of the 10 MSOME parameters: normalcy of spermatozoa - 0.96; normalcy of sperm nucleus - 0.86; normalcy of chromatin -0.87; normalcy of nuclear shape - 0.88; small nucleus -.96; large nucleus-0.72; short nucleus- 0.86; narrow nucleus- 0.90; wide nucleus- 0.89. The only MSOME parameter with a low Cronbach's alpha was nuclear regional disorder (0.61).

Intertechnician variability was not assessed because the entire study was performed by one observer (B.B.).

### Statistical analysis

Data are presented as means ± SE. Statistical evaluation was performed with the SPSSx package (Norusis, 1985). Pearson's correlation analysis was used to identify relationships between MSOME characteristics and ICSI outcome, and analysis of variance was used to compare sperm samples with decreased and normal fertilization rates and samples from males who did and did not achieve pregnancy. Nonparametric statistics - Kruskal-Wallis test followed by Mann-Whitney test - were performed concomitant to avoid possible artifacts due to the high standard deviations of some of the sperm parameters. The nonparametric methods yielded results similar to those reported here.

The receiver operating characteristic (ROC) curve analysis was used to determine the prognostic accuracy of each MSOME characteristic as well as its ability to correctly classify subjects into decreased and normal fertilization subgroups and successful and failed pregnancy subgroups. The sperm morphological parameters were analyzed for diagnostic sensitivity and specificity, and the threshold for each analyzed parameter was calculated for optimal sensitivity and specificity. The area under the ROC curve was expressed as a percentage.

### RESULTS

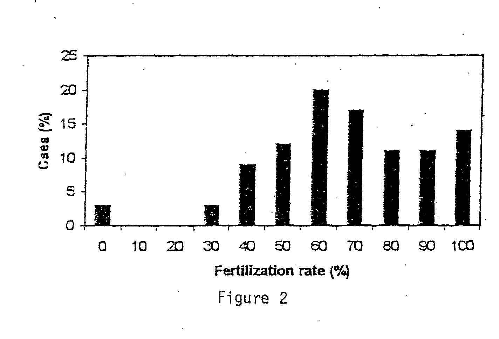

A total of 1074 oocytes were retrieved. Sperm injection was performed in 908 mature oocytes (85%), and fertilization occurred in 591 (65%). The mean fertilization rate per case was 64.2±2.2% (median 62.4%; Fig. 2). No statistically significant correlation was observed between the fertilization rate following ICSI and the number of retrieved or injected ova, or between the fertilization rate and the age of the male or female partners.

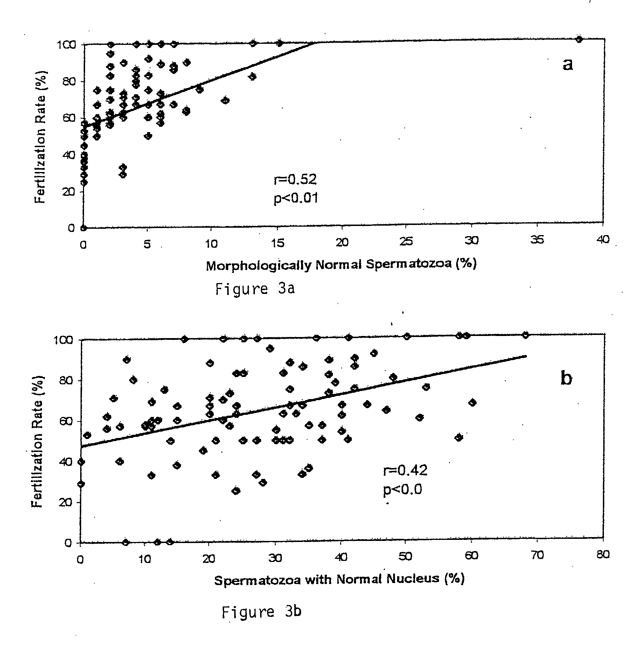

On MSOME, the mean incidence of morphologically normal spermatozoa in the samples examined was 3.3±0.5% (median 2.5%; range 0-38%). Only one male exhibited the extreme value of 38% for this MSOME parameter; in the other 99 patients, the incidence was 0-15%. The incidence of morphologically normal spermatozoa was found to have a positive and significant correlation with the fertilization rate following ICSI (r=0.52, p<0.01; Fig. 3a); this correlation held true even when the patient with 38% normal spermatozoa was excluded. Division of the patients by fertilization rate as presented in Table II hereinafter, showed that the subgroup with a low fertilization rate (<60%, n=47) exhibited a significantly lower incidence of the morphologically normal spermatozoa than the subgroup with a normal fertilization rate (>60%, n=53) (1.3±0.3% vs 5.8±0.6, F=28.2, p<0.01).

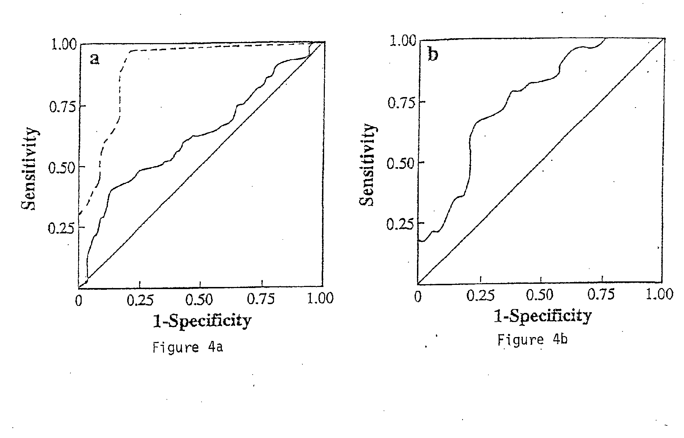

None of the 24 males with 0% morphologically normal spermatozoa in the sperm motile fraction exhibited a normal fertilization rate (mean 38.7±2.3%; Fig. 3a). Using ROC curve analysis, we demonstrated that the incidence of spermatozoa with normal morphology observed by MSOME had a very high predictive value for fertilization rate following ICSI (area under ROC curve: 88%, cut-off value: 2.5%, optimal sensitivity: 81%, optimal specificity: 78%; as shown in Table III hereinafter and in Fig. 4a.

**Table II.**

| Comparison of normal and low fertilization subgroups and of successful and failed pregnancy subgroups by significant MSOME parameters. | | | | |
|---|---|---|---|---|
| **MSOME Parameters** | **Subgroups** | | | |
| | **Fertilization** | | **Pregnancy** | |
| | **Normal (n=53)** | **Decreased (n=47)** | **Successful (n=28)** | **Failed (n=67)** |
| **Normal spermatozoa** | **5.8±0.6** | **1.3±0.3**^{**a**} | 4.6±4.1 | 3.6±3.3 |
| **Normal nucleus** | **31.9±2.8** | **21.7±2.2**^{**a**} | **34.3±1.8** | **24.5±1.7**^{**a**} |

| | | | | |
|---|---|---|---|---|
| ^{a}Significant difference (p≤0.01) | | | | |

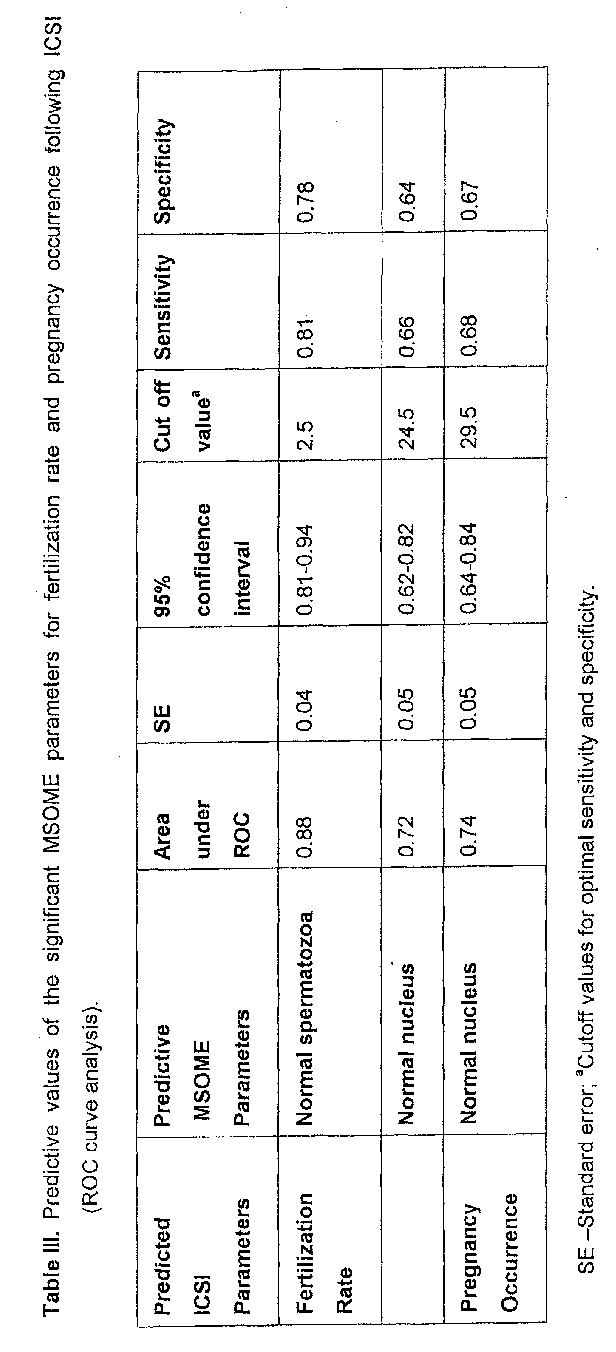

Of the six sperm subcellular organelles examined by our method, only the morphological normalcy of the sperm nucleus (chromatin + shape) correlated significantly with the fertilization rate (r=0.42, p<0.01; Fig. 3b). The mean incidence of this sperm parameter was 26.8±1.7% (median 25.0%, range 0-68%). It was able to significantly differentiate the normal and low fertilization subgroups (F=11.2, p<0.01; Table II), and had predictive value for the ICSI fertilization rate (area under ROC curve: 72%; cut-off value: 24.5%; optimal sensitivity: 66%; optimal specificity: 64%; Table III; Fig. 4a). None of the statistical methods revealed any significant association between the ICSI fertilization rate and the other MSOME parameters detailed in Table I.

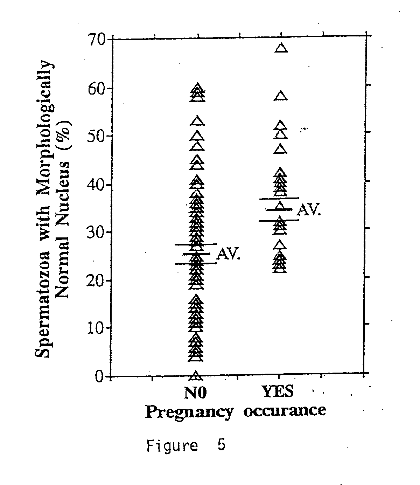

Twenty-eight pregnancies were achieved during this study. In 5 cases no embryos were transferred; therefore, the mean pregnancy rate per transfer was 29.5±1.6%. No significant correlation was observed between the achievement of pregnancy and any of the following parameters: number of retrieved oocytes, number of injected ova, fertilization rate, age of the male partner, or age of the female partner. There was also no statistically significant correlation between the pregnancy rate and the incidence of morphologically normal spermatozoa observed by MSOME. Of the six sperm subcellular organelles examined, only the morphological normalcy of the sperm nucleus (chromatin + shape) correlated significantly with achievement of pregnancy (r=0.38, p <0.01). A comparison of the patients who did and did not achieve pregnancy revealed that this sperm parameter was able to significantly differentiate between the successful and failed subgroups (n=28 and n=67, respectively, F=13.1, p<0.01; Table II). It is noteworthy that no patient exhibiting less than 20% spermatozoa with a morphologically normal nucleus achieved pregnancy (Fig. 5). ROC curve analysis demonstrated that normalcy of the sperm nucleus had predictive value for pregnancy occurrence (area under ROC curve: 74%; cut-off value: 28.5%; optimal sensitivity: 68%; optimal specificity: 67%; Table III; Fig. 4b). None of the statistical methods revealed any significant association between pregnancy rate and the other MSOME parameters detailed in Table I.

### DISCUSSION

The assessment of the possible association between male fertility potential and sperm morphology in humans is very complicated mainly for two reasons: a. Human spermatozoa exhibit a high structural variability. b. Clinicians cannot be sure that the sperm cells randomly selected for examination indeed represent those that are capable of fertilizing the ova. The first problem can be solved by morphological classification of the many specific sperm cell malformations according to the separate subcellular organelles, as shown in our earlier study [(Bartoov,B.,Fisher,J., Eltes,F., *et al.,* 1981 A comparative morphological analysis of abnormal human spermatozoa. In Insler, V. and Bettendorf,G. (eds), *Advances in Diagnosis and Treatment of Infertility*. Elsevier, Amsterdam, north Holland, pp. 355-373.)] The morphological state of the sperm organelles defined by this method was found to have the highest predictive ability for male fertility potential (Bartoov et al., 1990, 1994 ibid). The second difficulty can be solved in our era of IVF-ICSI by morphologically examining only the most active spermatozoa from the sperm fraction selected for microinjection. The new MSOME approach combines both these solutions. Using a high power light microscope equipped with Nomarski optics, we were able to perform a morphological analysis at the subcellular level in real time of the most active spermatozoa selected for ICSI.

The results of this study disagree with the current, widely accepted opinion that neither the type nor the extent of sperm impairment has an important influence on the outcome of ICSI, as mentioned earlier. The application of MSOME to the sperm fractions selected for micromanipulation illustrated that sperm morphological malformations have a negative effect on ICSI fertilization and pregnancy rates. Of the six sperm subcellular organelles examined, a morphologically normal sperm nucleus proved to be the most important sperm parameter influencing ICSI outcome. One explanation for our finding is the possible ability of MSOME to reflect nuclear DNA content and organization, which have been reported to exert a very significant effect on ICSI fertilization rate and embryo development.

We found that spermatozoa free of any specific morphological malformations exhibited the highest positive correlation with ICSI fertilization rate. This finding is compatible with the studies of (Mercan,R., Lanzendorf,S.E., Mayer,J.Jr., *et al* (1998) The outcome of clinical pregnancies following intracytoplasmic sperm injection is not affected by semen quality. Andrologia, 30, 91-95; and Osawa,Y., Sueoka,K., Iwata,S., *et al* (1999) Assessment of the dominant abnormal form is useful for predicting the outcome of intracytoplasmic sperm injection in the case of severe teratozoospermia. *J. Assist. Reprod. Genet., 16, 436-442.*), who reported a slightly reduced fertilization rate in cases of low incidence of morphologically normal spermatozoa. According to our data, at least 50% of the males included in ICSI programs exhibit a low (< 2.5%) incidence of morphologically normal motile spermatozoa, even when post-routine, pre-selection with MSOME was applied, which may explain the mean fertilization rate of 65% reported by many IVF centers. The significant association of the normalcy of the sperm cell with the fertilization rate, but not with the achievement of pregnancy, may be explained by the very narrow range of distribution (0-15%) of this parameter, making it insufficiently sensitive to differentiate the failed pregnancy subgroup from the successful one, which numbered only 28 patients. By contrast, the normalcy of the sperm nucleus exhibited a very wide range (0-68%). Therefore, this sperm parameter serves as a valuable predictive tool for both ICSI fertilization and pregnancy outcome.

We are aware that the prediction value and the optimal sensitivity and specificity values of the normal sperm nucleus for pregnancy occurrence were relatively low (0.74%, 68% and 67%, respectively). These findings were probably attributable to the wide, random ICSI population selected for this prospective study, which may have included patients with egg factor or other female factor infertility. Nevertheless, the correlation of normalcy of sperm nuclei with ICSI pregnancy outcome was significant. This emphasizes, in our opinion, the role of this morphological sperm parameter in ICSI success. Furthermore, it confirms our previous study showing the importance of the normalcy of the sperm head subcellular organelles in achieving pregnancy by both IVF and ICSI (Berkovitz et al., 1999 ibid).

The selection of spermatozoa for microinjection by the embryologist is not as efficient as natural sperm selection. Therefore, if the normalcy of the sperm nucleus is, indeed, strongly correlated with ICSI outcome, lower pregnancy rate would be expected for ICSI than for IVF. Actually, they are reported to be very similar. This discrepancy may be explained by our previous finding that only ICSI and not conventional IVF is able to overcome sperm tail abnormalities (Berkovitz et al., 1999).

We believe this advantage of ICSI together with the exclusive injection of sperm cells with normal nucleus, as determined by MSOME, may make ICSI the most effective assisted reproduction technique with respect to both outcome and cost. Indeed, in a preliminary study, it has now been found an over eight fold improvement of the pregnancy rate in cases that previously failed in at least two previous consecutive IVS-ICSI cycles when sperm cells with normal nuclei according to MSOME, were injected into the ovum by micromipulation. This new method has now been tentatively named Intra Cytoplasmic Preselected Sperm Injection (IVF-IPSI). The new methodology will significantly improve ICSI pregnancy outcome. The switch to MSOME selection is not particularly complicated, as it uses the same laboratory configuration as the routine IVF-ICSI procedure, namely, observation of motile spermatozoa in a droplet under oil with an inverted microscope. By simply upgrading the light microscope already present in the IVF laboratory with a X100 objective lens, enhanced by digital imaging, the embryologist will be able to sort out sperm cells with a normal nucleus

It will be evident to those skilled in the art that the invention is not limited to the details of the foregoing illustrative embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A method for individually selecting spermatozoa for intracytoplasmic sperm microinjection comprising effecting a real-time morphological evaluation of washed, fresh, motile spermatozoa without fixation using a high power light microscope having a magnification of at least x4000, selecting individual spermatozoon exhibiting high normalcy values for the morphological state of the nuclei of each individual spermatozoon and retrieving selected individual motile spermatozoon with nuclei exhibiting a smooth oval-shaped configuration, a length of between about 4.47 and 5.03 µm, and a width of about 3.08 and 3.48 µm.

2. A method according to claim 1, wherein said retrieved spermatozoon are further **characterized by** a nuclear chromatin content of less than 4 per cent vacuoles for the entire nuclear area.

3. A method according to claim 1, further comprising evaluating the normalcy of the acrosome, postacrosomal lamina, neck, mitochondria, tail and nucleus subcellular organelles of each individual spermatozoon.

4. A method according to claim 1, wherein said retrieval is effected utilizing a micromanipulated transparent needle for aspirating said selected individual motile spermatozoon.

5. A method according to claim 4, wherein said needle has a diameter of between about 15 and 20 microns.

6. A method according to claim 4, wherein said needle is made of glass.
